# EUROPEAN PATENT SPECIFICATION

(11) **EP 3 528 698 B1**
(45) Date of publication and mention of the grant of the patent: **12.08.2020**
(21) Application number: 17794911.2
(22) Date of filing: 19.10.2017
(51) Int. Cl.: A61B 5/0205, A61B 5/00, A44C 5/14

(54) **WEARABLE SENSING DEVICE**
ERFASSENDE WEARABLE-VORRICHTUNG
DISPOSITIF DE DÉTECTION POUVANT ÊTRE PORTÉ

(30) Priority: 20.10.2016 NL 2017648
(43) Date of publication of application: 28.08.2019
(73) Proprietor: Livassured B.V., 5261 AD Vught (NL)
(72) Inventor: TIELENS, Theo Ary Asmund, 5261 AD Vught (NL); HAAN, DE, Michiel, 5261 AD Vught (NL)
(74) Representative: Renkema, Jaap
(86) International application number: PCT/EP2017/076752
(87) International publication number: WO 2018/073364

(56) References cited:
- EP-A2- 2 612 595
- FR-A1- 2 686 713
- US-A1- 2010 175 232
- US-A1- 2014 084 033

## Description

### FIELD OF THE INVENTION

The invention relates to a wearable sensing device and a method of attaching an elastic band to the wearable sensing device.

### BACKGROUND

Wearable sensor devices are known for monitoring physiological parameters such as heart rate, skin resistance, temperature, etcetera. Such devices can be worn on various body parts and limbs. Wearable sensor devices can for example be worn on the wrist. In other applications sensor devices may be worn on the upper arm.

Wearable sensor devices have a sensor which measures a physiological parameter. An example of such a sensor is a photoplethysmographic sensor, which measures a heart rate using light transmitted into a subject's skin. Light from underlying tissue is reflected to a photosensitive sensor. The amount of reflected light depends on blood pressure. While the blood pressure varies in time with the subject's heart rate, the amount of reflected light thus allows the heart rate to be established accurately.

The accurately established heart rate can be used for monitoring the heart directly, or for establishing parameters derived from the heart rate. An example is epileptic seizure detection, wherein a sudden heart rate change is indicative for the presence of a seizure. Such detection may be combined with other parameters such as limb motion, posture or position to obtain a more accurate detection or reduce false detection.

In a cases wherein sensor pressure is of importance, accurate (re)positioning and fastening of the wearable sensor device is essential. At the same time wearing comfort should be maintained and health hazard from pinching or squeezing is to be kept low. As there is great variation in limb or arm diameter a great variation in band length is be required to accommodate a large variety of users. Commonly elastic bands or straps are used to achieve this. Straps, however, require clasps and do not provide sufficient comfort, flexibility and pressure accuracy during the period the sensor device is worn. Elastic band is an alternative, however, the variety in limb diameter is too great to accommodate all possible subjects. Attaching elastic band to the wearable device however may cause inaccuracy in the pressure applied to the device, since normally the band is fastened by folding it over a bar of the device and since then the elasticity modulus is not uniform, which causes the band to be non-uniformly stretched over its length. Examples of fastening mechanisms for wearable devices can be found in US 2014/0084033 A1 and EP 2 612 595 A2.

Thus it is an object of the invention to provide for a wearable sensor device which can be easily adapted for every wearer to provide accurate pressure control and comfort, and having a uniform elasticity modulus over the entire length of the band.

### SUMMARY

The object is achieved in a wearable sensing device, comprising a sensor carrier having a sensor at a lower side for touching the wearers skin, an elastic band having two ends, a fastener attached to each end of the band, two elongated fastening slots extending from a top side opposite to the lower side of the sensor carrier, wherein the elongated fastening slots are located at opposite locations near an edge of the sensor carrier. The elongated fastening slots are arranged for removably receiving the fasteners. The fasteners are removably insertable into the fastening slots. The lower side having the sensor is the side of the sensor device to be pressed against the subject's skin. The top side is the visible side of the sensor device and is exposed to the environment. The lower side may contact the skin of a limb, e.g. an arm of the subject wearing the device, allowing it to perform its measurements.

By using fasteners, the elastic band can be used in one single piece without any folds and/or double layers. This ensures the uniform elasticity modulus to be maintained over the entire length of the elastic band. Using fasteners which are introduced into the top side of the sensor carrier allows a controlled pressure to be exerted in the direction of the lower side, the side that is in contact with the subject's skin.

In an embodiment, each fastener has an oblong shaped body, having an elongated slot for receiving the elastic band's end.

This allows the elastic band's end to be pulled through the fastener, which in turn allows the elastic band to be connected to the sensor device indirectly through the fasteners.

In an embodiment, the fastener body has a rim and an edge. The rim ensures that the fastener remains on top of the top side of the sensor device, and prevents it from falling through the fastenings slots thereof. The edge is designed to cooperate with a corresponding edge inside each fastening slot, for creating a snap fit retention. For this purpose, the fastener is made of resilient material which allows the fastener to be compressed for releasing from the snap fit. Thus involuntary release of the fasteners from the fastenings slots is prevented, while at the same time the fasteners can be simply released from the fastenings slots when required by the user without undue effort.

In an embodiment, each elongated fastening slot has a guide element and an edge for catching the edge of the fastener body.

In an embodiment, each fastener has retention means within its elongated slot for retaining the band's end.

In an embodiment, the retention means comprise a plurality of teeth extending from each of the walls in a direction to the wall opposite of the elongated slot. The retention means can also be hooks, which engage the elastic band when it is inserted into the elongated slot of the fastener. By giving the retention means a single directed action, it causes the elastic band to be secured in one direction, while it can slide further into the elongated slot of the fastener in the opposite direction. Thus fine tuning of the elastic band length can be achieved.

The object is further achieved in a method of attaching an elastic band to a wearable sensing device according to any of the preceding claims, the method comprising measuring a circumference of a limb or body part at which the device is to be worn, cutting the elastic band in a single piece having a length greater than measured circumference, sliding a fastener over each end of the single piece of elastic band, inserting one of the fasteners in a corresponding fastening slot of the device, adjusting the position of at least one of the fasteners relative to its corresponding end of the elastic band, and finally trimming the corresponding end of the elastic band using a cutting means.

### BRIEF DESCRIPTION OF THE DRAWINGS

Fig. 1 shows a wearable sensor device according to an embodiment of the invention.
Fig. 2 shows a wearable sensor device according to an embodiment of the invention.
Fig. 3 shows a wearable sensor device according to an embodiment of the invention.
Fig. 4 shows a diagram of a fastener of the wearable sensor device according to an embodiment of the invention.
Fig. 5 shows a diagram of a cross section of a fastener of the wearable sensor device according to an embodiment of the invention.
Fig. 6 shows a detail of a wearable sensor device according to an embodiment of the invention.

### DETAILED DESCRIPTION OF EMBODIMENTS

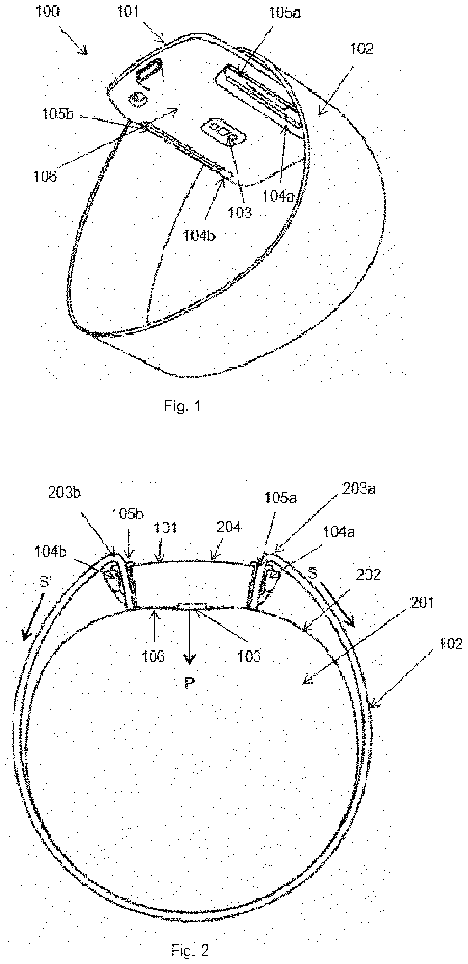

Figure 1 shows an wearable sensor device 100 having a sensor carrier 101 with an elastic band 102 attached thereon. The sensor carrier 101 has a sensor 103 at the bottom surface 106. The bottom surface 106 is the surface of the sensor carrier 101 facing the subject's skin. The position of the fixation of the elastic band 102 insures that the sensor carrier 101 is pressed against the subject's skin. At the bottom surface 106 fastening slots 104a, 104b can be observed, wherein fasteners 105a, 105b can be introduced which attach end of the elastic band 102 to the sensor carrier 101.

The sensor carrier 101 can be fabricated from a plastic, metal or any material suitable for housing a sensor and its electronic circuitry including a battery required for operation of the sensor 103. The sensor carrier 101 may furthermore accommodate controls such as switches to operate the sensor 103. Moreover, the sensor carrier may comprise visual or acoustic alarms or displays.

The elastic band 102 can be made from textile fibers, which may be supplemented with elastomer fibers, or from elastic textile fibers alone.

In figure 2 a cross section of the subject's limb 201 is shown having a an wearable sensor device 100 attached to it. The sensor carrier 101 is shown positioned with its bottom surface 106 pressed against the subject's skin 202. The fasteners 105a,105b are shown having ends of the elastic band 102 guided there through. The fasteners 105a, 105b are inserted in the fastening slots 104a, 104b on the upper, top side 204 of the sensor carrier 101. From fig. 2 it is clear that the elastic band 102 emanates from the fastener 105a and bends around the arm 201 starting at tipping point 203a. On the other side, the band 102 bends back at tipping point 203b into fastener 105b. It will be clear from fig. 2 that a pulling force S,S' in the elastic band 102 cause a pressure P to be exerted at the surface 106, and more specifically at the sensor 103 in contact with the subject's skin 202.

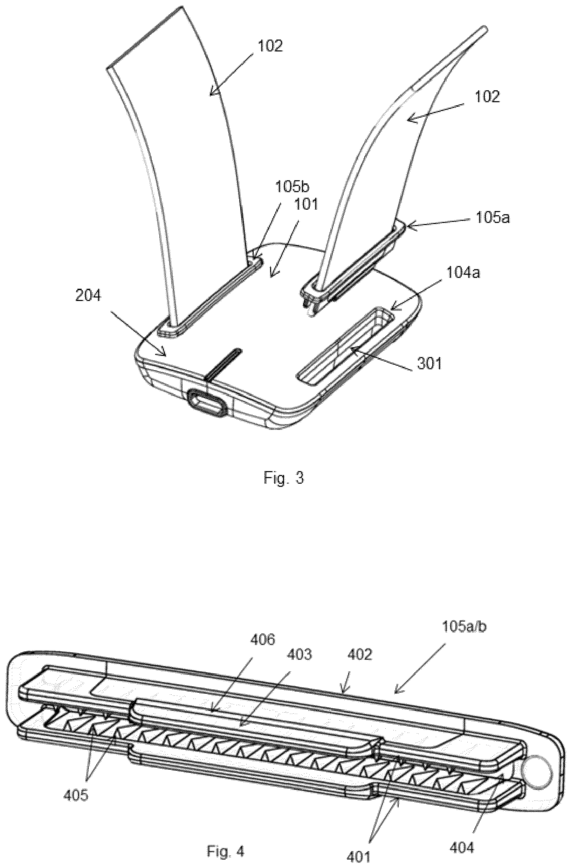

In figure 3 the sensor carrier 101 is shown having the band 102 attached to the fastener 105b inserted in fastening slot 104b (not shown) in the top surface 204 of the sensor carrier. In fig. 3 another end of the elastic band 102 is shown inserted through fastener 105a, which is shown taking out from fastening slot 104a. Within the fastening slot 104a, a retention edge 301 is shown for retention of the fastener 105a.

Figure 4 shows a fastener 105a/b having walls 401 arranged adjacent an elongated slot 404, for guiding through an end of the elastic band 102. At least one of the walls 401 is provided with a guide element 403 which guide element 403 has an edge 406, for cooperating with a corresponding retention edge 301 in the fastening slot 104a, 104b of the sensor carrier 101. In fig. 4 both walls 401 are provided with a guide element (403). The walls 401 are provided with teeth 405 at a side of each wall facing the other wall 401 opposite the slot 404, which provide for retention of the end of the elastic band 102 inserted through the elongated slot 404. The teeth extend through the elastic band surface and retain the elastic band by deforming the band and/or by friction, and/or by engaging fibers in the elastic band. As an alternative for teeth, hooks may also be used.

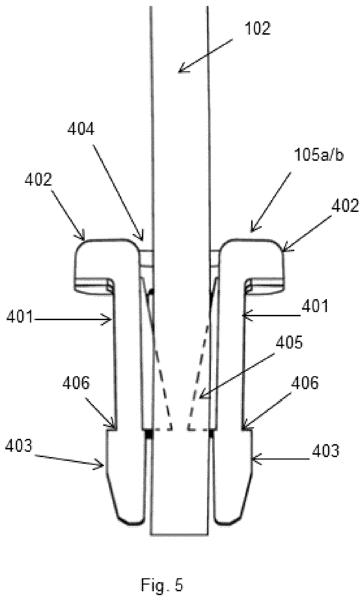

In figure 5 a cross section of the fastener 105a/b is shown with the elastic band 102 extending through the elongated slot 404 and being retained by the teeth 405, which are arranged opposite each other on respective walls 401. Furthermore in fig. 5 the guide elements 403 are shown extending from the walls 401 having an edge 406 for retention in the fastening slots 104a, 104b.

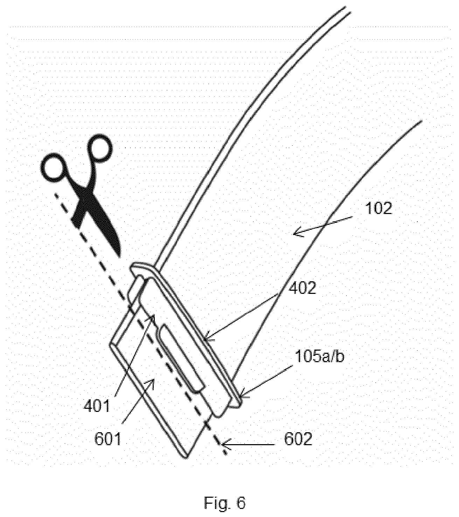

In figure 6 the fastener 105a/b is shown having an end of the elastic band 102 extending through the fastener, showing that an unused part 601 of the elastic bands end can be trimmed off at trimming line 602 just clear from the guide element 403 to obtain an appropriate length. The total length of elastic band 102 can be determined by measuring a circumference of the subject's limb, or the determined tentatively by testing the sensor carrier pressure P against the subject's skin 202, or by testing the pulling force S,S' of the elastic band 102 as shown in figure 2. The trimming can also be performed when the fastener 105a, 105 b is inserted in the corresponding fastening slot 104a, 104b.

In the examples described above, the elastic band is attached to the sensor carrier via two fasteners 105a, 105b, by inserting the fasteners 105a, 105b into corresponding fastenings slots 104a, 104b . It can be considered to permanently attach one end of the elastic band to the top side 204 of the sensor carrier 101, while the opposite end of the elastic band 102 is attached with a fastener 105a or 105b in a corresponding fastening slot 104a or 104b of the sensor carrier 101.

The oblong shape of the fastener in combination with the elongated fastening slot may also be constructed with mutually corresponding shaped forms, such as round, oval or square.

### REFERENCE NUMERALS

- 100: wearable sensing device
- 101: sensor carrier
- 102: band
- 103: sensor
- 104a, 104b: receiving slot
- 105a, 105b: fastener
- 106: bottom surface
- 201: arm
- 202: skin
- 203a, 203b: tipping point
- 204: top side of the sensor carrier
- 301: retention edge
- 401: wall
- 402: rim
- 403: guide
- 404: slot
- 405: teeth
- 406: edge
- 601: unused part
- 602: trimming line

## Claims

1. Wearable sensing device (100), comprising:
• a sensor carrier (101) having a sensor (103) at a bottom surface (106) of the sensor carrier (101), for pressing said sensor (103) against a subject's skin;
• an elastic band (102) having two ends;
• two fasteners (105a, 105b), each fastener (105a, 105b) attached to opposite ends of the elastic band;
• two elongated fastening slots (104a, 104b) extending from a top side (204) to said bottom surface (106) of the sensor carrier (101), wherein the elongated fastening slots are located at opposite locations near an edge of the sensor carrier (101), wherein the elongated fastening slots (104a, 104b) are arranged for removably receiving the fasteners (104a, 104b);
• **characterised in that** in use, the fasteners (105a,105b) are inserted in the fastening slots (104a,104b) on the top side (204) of the sensor carrier (101), and wherein said elastic band (102) emanates at one end from a first one of said two fasteners (105a) for bending around a limb or body part (201) of said subject, starting at a first tipping point (203a) at said top side (204) of said sensor carrier (101), and wherein at an opposite end of said the band 102, said band (102) bends back at a second tipping point (203b) at said top side (204) of said carrier
(101) into a second one of said two fasteners (105b).

2. Device according to claim 1, wherein each fastener (105a, 105b) has an oblong shaped body comprising a rim (402), and walls (401) attached to the rim (402) having an elongated slot (404) arranged between the walls (401) for receiving the band's end.

3. Device according to claim 1 or claim 2, wherein at least one of the walls is provided with a guide element (403) having an edge (406).

4. Device according to claims 2 or 3, wherein each elongated fastening slot has a retention edge (301) for receiving the edge (406) of the fastener body for creating a snap fit retention.

5. Device according to any of the preceding claims, wherein each fastener has retention means (405) within its elongated slot for retaining the band's end.

6. Device according to claim 5, wherein the retention means (405) comprise a plurality of teeth extending from the walls (401) in a direction to wall (401) opposite of the elongated slot (404).

7. Device according to any one of the preceding claims, wherein said elastic band (102) has a tunable length between said fasteners (105a, 105b).

8. Method of attaching an elastic band to a wearable sensing device according to any of the preceding claims, the method comprising:
• measuring a circumference of a limb or body part (201) at which the device (101) is to be worn;
• cutting the elastic band (102) in a single piece having a length greater than measured circumference;
• sliding a fastener (105a, 105b) over each end of the single piece of elastic band;
• inserting one of the fasteners in a corresponding fastening slot (104a, 104b) of the device (101);
• adjusting the position of at least one of the fasteners (105a, 105b) relative to its corresponding end of the elastic band (102);
• trimming the corresponding end of the elastic band (102) using a cutting means.

## Patentansprüche

1. Tragbare Sensorvorrichtung (100), umfassend:
• einen Sensorträger (101) mit einem Sensor (103) an einer Bodenfläche (106) des Sensorträgers (101) zum Andrücken des Sensors (103) an die Haut einer Person;
• ein elastisches Band (102) mit zwei Enden;
• zwei Befestigungsmittel (105a, 105b), wobei jedes Befestigungsmittel (105a, 105b) an gegenüberliegenden Enden des elastischen Bandes angebracht ist;
• zwei längliche Befestigungsschlitze (104a, 104b), die sich von einer Oberseite (204) zu der Bodenfläche (106) des Sensorträgers (101) erstrecken, wobei die länglichen Befestigungsschlitze an gegenüberliegenden Stellen nahe einer Kante des Sensorträgers (101) angeordnet sind, wobei die länglichen Befestigungsschlitze (104a, 104b) zur trennbaren Aufnahme der Befestigungsmittel (104a, 104b) angeordnet sind;
• **dadurch gekennzeichnet, dass**
wenn in Gebrauch, die Befestigungsmittel (105a, 105b) in die Befestigungsschlitze (104a, 104b) auf der Oberseite (204) des Sensorträgers (101) eingesetzt sind, und wobei das elastische Band (102) an einem Ende von einem ersten der beiden Befestigungsmittel (105a) zum Biegen um ein Glied oder einen Körperteil (201) der Person ausgeht, beginnend an einem ersten Kipppunkt (203a) an der Oberseite (204) des Sensorträgers (101), und wobei an einem entgegengesetzten Ende des Bandes (102) das Band (102) sich an einem zweiten Kipppunkt (203b) an der Oberseite (204) des Trägers (101) in ein zweites der beiden Befestigungsmittel (105b) zurück biegt.

2. Vorrichtung nach Anspruch 1, wobei jedes Befestigungsmittel (105a, 105b) einen länglichen Körper mit einem Rand (402) umfasst und an dem Rand (402) befestigte Wände (401) mit einem länglichen Schlitz (404) aufweist, der zwischen den Wänden (401) zur Aufnahme des Endes des Bandes angeordnet ist.

3. Vorrichtung nach Anspruch 1 oder Anspruch 2, wobei mindestens eine der Wände mit einem Führungselement (403) versehen ist, das eine Kante (406) aufweist.

4. Vorrichtung nach Anspruch 2 oder 3, wobei jeder längliche Befestigungsschlitz eine Haltekante (301) zur Aufnahme der Kante (406) des Befestigungskörpers aufweist, um einen Arretierungs-Schnappverschluss zu schaffen.

5. Vorrichtung nach einem der vorhergehenden Ansprüche, wobei jedes Befestigungsmittel Haltemittel (405) in seinem länglichen Schlitz aufweist, um das Bandende zurückzuhalten.

6. Vorrichtung nach Anspruch 5, wobei die Haltemittel (405) eine Vielzahl von Zähnen umfassen, die sich von den Wänden (401) in einer Richtung zur Wand (401) erstrecken, die dem länglichen Schlitz (404) gegenüberliegt.

7. Vorrichtung nach einem der vorhergehenden Ansprüche, wobei das elastische Band (102) eine abstimmbare Länge zwischen den Befestigungsmitteln (105a, 105b) aufweist.

8. Verfahren zum Befestigen eines elastischen Bandes an einer tragbaren Sensorvorrichtung nach einem der vorhergehenden Ansprüche, das Verfahren umfassend:
• Messung eines Umfangs eines Gliedes oder Körperteils (201), an dem die Vorrichtung (101) getragen werden soll;
• Schneiden des elastischen Bandes (102) in ein einzelnes Stück mit einer Länge, die größer als der gemessene Umfang ist;
• Schieben eines Befestigungsmittels (105a, 105b) über jedes Ende des einzelnen Stücks aus elastischem Band;
• Einfügen eines der Befestigungsmittel in einen entsprechenden Befestigungsschlitz (104a, 104b) der Vorrichtung (101);
• Einstellen der Position mindestens eines der Befestigungsmittel (105a, 105b) relativ zu seinem entsprechenden Ende des elastischen Bandes (102);
• Trimmen des entsprechenden Endes des elastischen Bandes (102) mit einem Schneidemittel.

## Revendications

1. Dispositif de détection portatif (100) comprenant :
- un support de capteur (101) comportant un capteur (103) au niveau d'une surface inférieure (106) du support de capteur (101), destiné à presser ledit capteur (103) contre la peau d'un utilisateur ;
- une bande élastique (102) comportant deux extrémités ;
- deux éléments de fixation (105a, 105b), chaque élément de fixation (105a, 105b) étant relié à des extrémités opposées de la bande élastique ;
- deux fentes de fixation allongées (104a, 104b) s'étendant à partir d'un côté supérieur (204) vers ladite surface inférieure (106) du support de capteur (101), les fentes de fixation allongées se trouvant à des emplacements opposés à proximité d'un bord du support de capteur (101), les fentes de fixation allongées (104a, 104b) étant conçues pour recevoir les éléments de fixation (104a, 104b) de façon amovible ;
**caractérisé en ce que**
- pendant l'utilisation, les éléments de fixation (105a, 105b) sont insérés dans les fentes de fixation (104a, 104b) sur le côté supérieur (204) du support de capteur (101), et dans lequel ladite bande élastique (102) part à une extrémité d'un premier parmi les deux éléments de fixation (105a) pour contourner une partie de membre ou de corps (201) dudit utilisateur, à partir d'un premier point charnière (203a) au niveau dudit côté supérieur (204) dudit support de capteur (101), et dans lequel, à une extrémité opposée de ladite bande (102), ladite bande (102) est recourbée vers un deuxième point charnière (203b) au niveau dudit côté supérieur (204) dudit support (101), dans un deuxième parmi les deux éléments de fixation (105b).

2. Dispositif selon la revendication 1, dans lequel chaque élément de fixation (105a, 105b) comporte un corps oblong comprenant un rebord (402), et des parois (401) reliées au rebord (402) présentant une fente allongée (404) disposée entre les parois (401) pour recevoir l'extrémité de la bande.

3. Dispositif selon la revendication 1 ou la revendication 2, dans lequel l'une au moins des parois est dotée d'un élément de guidage (403) comportant un bord (406).

4. Dispositif selon la revendication 2 ou 3, dans lequel chaque fente de fixation allongée comporte un bord de retenue (301) destiné à recevoir le bord (406) du corps d'élément de fixation pour créer une retenue par encliquetage.

5. Dispositif selon l'une quelconque des revendications précédentes, dans lequel chaque élément de fixation comporte un moyen de retenue (405) à l'intérieur de sa fente allongée, pour retenir l'extrémité de bande.

6. Dispositif selon la revendication 5, dans lequel le moyen de retenue (405) comprend une pluralité de dents s'étendant à partir des parois (401) dans une direction vers la paroi (401) opposée à la fente allongée (404).

7. Dispositif selon l'une quelconque des revendications précédentes, dans lequel ladite bande élastique (102) présente une longueur réglable entre lesdits éléments de fixation (105a, 105b).

8. Procédé de fixation d'une bande élastique sur un dispositif de détection portatif selon l'une quelconque des revendications précédentes, le procédé comprenant :
- la mesure d'une circonférence d'une partie de membre ou de corps (201) au niveau de laquelle le dispositif (101) doit être porté ;
- la découpe de la bande élastique (102) en une pièce unique présentant une longueur supérieure à la circonférence mesurée ;
- le glissement d'un élément de fixation (105a, 105b) par-dessus chaque extrémité de la pièce unique de bande élastique ;
- l'insertion de l'un des éléments de fixation dans une fente de fixation (104a, 104b) correspondante du dispositif (101) ;
- le réglage de la position de l'un au moins des éléments de fixation (105a, 105b) par rapport à son extrémité correspondante de la bande élastique (102) ;
- le raccourcissement de l'extrémité correspondante de la bande élastique (102) à l'aide d'un moyen de coupe.
